# EUROPEAN PATENT APPLICATION

(11) **EP 2 317 323 A1**
(43) Date of publication of application: **04.05.2011**
(21) Application number: 09306028.3
(22) Date of filing: 29.10.2009
(51) Int. Cl.: G01N 33/68

(54) **MALDI Mass Spectrometry (MALDI-MS) for proteins of high molecular weight**

(71) Applicant: Centre National de la Recherche Scientifique (CNRS), 75016 Paris (FR)
(72) Inventor: Fournier, Isabelle, 59830 Bourghelles (FR); Franck, Julien, 59260 Hellemmes (Lille) (FR); Longuespee, Rémi, 59199 Bruille Saint Armand (FR)
(74) Representative: Tetaz, Franck Claude Edouard

(57) **Abstract**

Methods for the detection of high molecular mass proteins in a biological tissue section by Matrix Assisted Laser Desorption Ionization Mass Spectrometry (MALDI-MS)

## Description

After 10 years of development, MALDI-MSI has become a powerful and versatile tool for analysing different classes of endogenous and exogenous molecules. The technology is being developed intensively for drugs imaging (1, 2). Molecular images of peptides and of polypeptides up to 25 kDa are now routinely obtained (3-7). More recently, major improvements have also been performed for imaging lipids (8-12). However, proteins exceeding 25 kDa are still not detected by MALDI MSI. This definitely represents a real technological bolt. In fact, certain class of proteins with important biological activities such as most of cytokines, growth factors, enzymes, receptors, proproteins, and neuropeptide precursors are larger than 20 kDa.

Detection of proteins of high molecular weight may require both specific detectors for detection of ions of high molecular mass and methods for efficient extraction/crystallization of high molecular weight proteins in the matrix.

MALDI-TOF apparatuses are generally equipped with MCP detectors based on the conversion of ions into electrons and subsequent amplification of electrons. However, this detection process is known to favour lower m/z because of the ion to electron conversion step. In fact, ion conversion coefficient varies as a negative exponential function according to the velocity of ions. In TOF analyzers, where ion velocity is inversely proportional to the square root of m/z ratio, higher m/z ions show a decreasing velocity with M increasing. Thus, in TOF with MCP detectors, higher molecular masses will be highly discriminated. This is a real limitation and it is clear that many signals are lost in the higher mass range. However, in classical MALDI applications such as in proteomics this is rarely a problem since complex mixtures of proteins with masses above 20°000 are not often analyzed. On the other hand, this has a dramatic effect in MALDI-MSI where one would need to collect information from higher mass proteins at the tissue section level in the context of a complex mixture. Use of new detectors based on the conversion of the ions into small ions prior to conversion into electrons seems to be a good alternative to MCP detectors to improve detecttion of high molecular masses. Such high mass detectors are for example described in WO2009/086642 and commercialized by COVALX AG.

Two recent studies describe matrix deposition techniques for the detection of high molecular mass proteins (13, 14). The first of these studies proposes extensive water washing procedures to deplete abundant soluble proteins from the tissue sections as well as automated application of matrix solutions containing a high percentage of organic solvent. This sample preparation allows the detection of a 28 kDa integral crystalline lens membrane protein (14). In the second approach, a variation of the sandwich matrix application protocol using Triton X-100 is shown to enable the detection of proteins ranging for m/z 25000 to 50000 (13).

In MALDI MS as well as in MALDI MSI, proteins are generally analysed using SA (sinapinic acid) in a polar solvent such as acetonitrile (AcN)/aqueous TFA (trifluoracetic acid) as matrix. However, with such preparation protocols if highly specific and resolved molecular images are obtained, only a restricted number of proteins in a restricted mass range are observed. Most of the proteins observed with reasonable signals are generally below 25°000-30°000 Da. From such observations different questions arise concerning the detection of higher mass proteins. One may assume that high mass proteins are not observed because they have different physico-chemical properties in terms of solubility and are not well extracted from the tissues by these classical solvents, or not well incorporated into matrix crystals during crystallization of the matrix.

For the detection of high molecular masses by MALDI-MSI, specific chemical treatments have been performed on the tissue sections prior to analysis. After chemical treatment with acetone, chloroform and EtOH, intense signals of proteins were detected with specific localization. It was already shown in previous studies that chemical treatments using organic solvents such as chloroform (18), or other solvents such as EtOH (19, 20) provide spectra with higher intensity and provide for detection of a larger number of proteins signals. However, even after such chemical treatments, protein signals above m/z 20°000 are very difficult or impossible to detect by MALDI-MSI. Only very few ions are present in this m/z range, in which some classes of proteins with interesting biological activities such as cytokines, growth factors, enzymes and receptors, should be observed.

Difficulties in analyzing high masses proteins are due to their distinct physico-chemical properties that lead to poor extraction and thus to co-crystallization with the matrix in the solvents used. In tissue analysis, the matrix is generally dissolved in polar solvents including AcN and aqueous TFA 0.1% as for classical MALDI sample preparation. AcN is a relatively hydrophobic solvent known to solubilize many proteins of intermediate hydrophobicity. Solvents such as HFIP (14, 21) or detergents such as Triton X100(13) were studied for direct analysis or MSI. Using these solvents different classes of molecules can be detected. In this context other procedures were investigated to improve extraction and ionization efficiency of high mass proteins for combination with high mass detection. In classical proteomics studies, procedures to extract proteins are generally more complicated and involve different steps to disrupt non covalent interactions and separate molecules form each other to improve their extraction. Apart from disulfide bridges, the main interactions are non covalent including ionic bonds, hydrogen bonds and hydrophobic interactions. These hydrophobic interactions are also responsible for some protein-protein interactions and for the bonding of lipids and other small apolar molecules to proteins. This may also explain why the detection of proteins directly on tissue sections after chemical treatment using chloroform is greatly improved (18). The best way to break these hydrophobic interactions is the use of solvents providing a hydrophobic environment. In polar solvents or pure water, a hydrophobic environment is created by the use of detergents such as Triton X100 or SDS (22) or of chaotropes such as urea (23). Detergents and chaotropes at high concentration change the properties of the solvent (dielectric constant, polarizability, etc...) and all the resulting interactions. For example, by decreasing hydrogen bonding of the solvent, the environment becomes immediately more suitable for an exposure of apolar groups and favors the dispersion of hydrophobic molecules and the unfolding of proteins (23). However, the use of chaotropes at high concentrations is not compatible with mass spectrometry especially in the imaging mode. It was also shown that the use of a basic pH could improve solubilization of proteins with an efficiency close to that of an extraction process using SDS (24). OG (octyl B-D-glucopyranoside) is a non ionic detergent that was previously shown to be compatible with MSI (25). Hydrophobic solvents such as HFIP (26) or TFE (27, 28) have been proposed for extraction of membrane proteins (29-31). TFE is already known for its use in high mass proteins extraction ( 32) in classical proteomics procedure and for its solubilisation properties for peptides and proteins (33). A solvent such as HFIP is also known for its extraction efficiency of hydrophobic proteins. However, protocols using these solvents for the extraction of high molecular mass proteins prior to MALDI-MSI have not been developed.

In the present application novel sample preparation methods have been developed for the detection of high molecular mass proteins by MALDI mass spectrometry. These methods are based on the use of apolar solvents including Hexafluoroisopropanol (1,1,1,3,3,3-Hexaluoro-2-propanol, HFIP) (15, 16) and 2,2,2-Trifluoroethanol (TFE) in association with sinapinic acid (SA) for integral membrane protein solubilisation. The proper extraction and incorporation of high mass proteins into the matrix crystals requires extraction, crystallization, re-solubilisation and a second crystallization step. Using this procedure, high mass proteins were detected with a high sensitivity even without the use of a high mass detector. However, when the chemical treatments were coupled with the use of high mass detector, detection of very high mass proteins up to m/z 110°000 with an extremely good intensity was performed.

Further, a method was developed to detect both successively small polar proteins and high molecular mass proteins. Small polar proteins were detected using classical MALDI matrices and after removal of this matrix, the sample preparation for high molecular mass detection was applied on the same sample. This method offers new perspectives for protein analysis and imaging from tissue sections by enabling both the detection of polar proteins and of higher mass proteins on the same tissue section or in the same sample.

These procedures were then tested in clinical application for the detection of proteins in the case of ovarian cancer. The results show that classical tissue preparation only lead to the observation of few proteins above m/z 20°000. On the other hand, after the new high mass treatment, a high number of proteins up to m/z 45°000 were easily detected with high intensity allowing for the direct observation of protein markers. This opens the door for new biomarker discovery and is comparable to what can be detected using 2D-Gel proteomic while providing valuable information on the localization of the protein. These treatments could easily be done on cancerous regions of interest established by the pathologist and validated by a hierarchical clustering processing. This work, enabling the detection of high mass proteins, opens the door to functional proteomic studies localizing proteins and their networking at tissue level. Both reporter peptides and antibodies may be tracked in tissues using the Tag-mass concept (17). Tracking of known proteins directly in tissue sections by mass spectrometry is now feasible.

### SUMMARY OF THE INVENTION

The present invention relates to a method for the detection of at least one protein in a biological sample by Matrix Assisted Laser Desorption Ionization Mass Spectrometry (MALDI-MS) comprising the following steps:
a) Applying a solution comprising an extraction solvent selected from TFE (2,2,2-trifluoroethanol) and HFIP (1,1,1,3,3,3-hexafluoro-2-propanol) onto the surface of the biological sample;
b) Applying a first MALDI matrix onto the surface of the biological sample and obtaining a first crystallized matrix on the surface of the biological sample;
c) Applying a second MALDI matrix onto the surface of the biological sample to solubilise the first crystallized matrix obtained in the previous step and re-crystallize both the first and the second matrix to obtain a crystallized matrix on the surface of the biological sample;
d) Ionization and desorption of proteins from the biological sample;
e) Analysis and detection of at least one protein by Mass Spectrometry.

Preferably, the first and second MALDI matrix are selected from SA (Sinapinic acid), 2,5-DHB (2,5-dihydroxybenzoic acid), SA/ANI (Aniline), SA/3AP (3-acetylpyridine), SA/DIENI (Diethylaniline) and SA/DANI (Dimethylaniline).

More preferably, the first and second MALDI matrix are SA (Sinapinic acid) matrices.

In a first embodiment, the solution applied in step a) comprises TFE, water and TFA (Trifluoracetic acid).

Preferably, the solution applied in step a) comprises between 20% and 80% TFE.

In preferred embodiments, step a) and step b) are performed simultaneously by applying a composition onto the surface of the biological sample comprising both HFIP and a first SA matrix.

Preferably, said composition applied onto the surface of the biological sample, comprises 20mg/ml SA in HFIP.

In one embodiment, the first MALDI matrix in step b) and/or the second MALDI matrix in step c) comprises SA, ethanol, water and TFA.

In another embodiment, the first MALDI matrix in step b) and/or the second MALDI matrix in step c) comprises SA, ACN (acetonitrile), water and TFA.

Most preferably, the first MALDI matrix in step b) comprises SA, ethanol, water and TFA and the second MALDI matrix in step c) comprises SA, ACN (acetonitrile), water and TFA.

Preferably, the first MALDI matrix and the second MALDI matrix comprise 20mg/ml SA.

Preferably, the biological sample is a biological tissue section.

Preferably, analysis and detection of at least one protein by Mass Spectrometry in step e) is performed by TOF (time of flight)-Mass Spectrometry.

The present invention also relates to a method for the detection of proteins in a biological sample by MALDI-MS comprising the following steps:
a) Applying a MALDI matrix onto the surface of the biological sample and obtaining a crystallized matrix on the surface of the biological sample;
b) Ionization and desorption of proteins from the biological sample;
c) Analysis and detection of proteins by Mass Spectrometry;
d) Removing the MALDI matrix applied in step a);
e) Detection of at least one protein in the same biological sample by Matrix Assisted Laser Desorption Ionization Mass Spectrometry (MALDI-MS) by a method as described above.

Preferably, in step a) the MALDI matrix is selected from from SA (Sinapinic acid), 2,5-DHB (2,5-dihydroxybenzoic acid), SA/ANI (Aniline), SA/3AP (3-acetylpyridine), SA/DIENI (Diethylaniline) and SA/DANI (Dimethylaniline).

More preferred, in step a) the MALDI matrix is a SA (Sinapinic acid) matrix.

Advantageously, steps a) to c) provide for the detection of at least one protein having a molecular weight below 20kDa and wherein steps d) to e) provide for the detection of at least one protein having a molecular weight above 20kDa.

Preferably, the MALDI matrix in step a) comprises SA, ACN, water and TFA.

Preferably, in step d) the MALDI matrix is removed in a bath of methanol and water.

### DETAILED DESCRIPTION OF THE INVENTION

Mass spectrometry is an analytical tool used for measuring the molecular mass of an analyte. Mass spectrometers can be divided into three fundamental parts, namely the ionization source, the analyzer and the detector.

One of the ionization methods used for the majority of biochemical analyses is MALDI (matrix assisted laser desorption ionization). MALDI is based on bombardment of sample molecules with a laser light to bring about sample ionization. A MALDI matrix is usually required to protect the sample and to facilitate ionization and desorption. The analyte or sample is embedded in this matrix.

Any analyser may be used in the methods of the present invention. Time-of-flight mass spectrometry is a common technique largely described in the litterature. TOF-MS is a method of mass spectrometry in which ions are accelerated by an electric field. The velocity of the ion depends on the mass-to-charge ratio. The time that it subsequently takes for the particle to reach a detector at a known distance is measured. This time will depend on the mass-to-charge ratio of the ion. From this time and the known experimental parameters one can find the mass-to-charge ratio of the ion.

In the methods of the present invention, the type of mass spectrometer advantageously used with MALDI is the TOF (time-of flight) mass spectrometer. However, any suitable mass spectrometer may be used in the methods of the present invention.

Various detectors may also be used in the methods of the present invention. Commonly used detectors for the detection of proteins include MicroChannel Plate detectors (MCP). Preferably, a high mass detector is used such as the COVALX™ detector described in WO2009/086642.

MALDI mass spectrometry imaging (MALDI-MSI) links the universal detection capability of mass spectrometry with the positional information of molecular histology, generating mass spectra correlated to known locations within a biological tissue. In MALDI imaging, tissue sections are mounted onto a support and a suitable MALDI matrix is applied to the tissue. The support is inserted into a MALDI mass spectrometer which records the spatial distribution of different analytes. Suitable image processing software is used to import data from the mass spectrometer to allow visualization of analytes on the optical image of the tissue section. MALDI-MSI has allowed researchers to directly image biomolecules such as proteins in biological tissue samples. This ability to image specific biological analytes is an important tool in many areas of research, such as determining the distribution and expression of specific proteins in healthy compared to diseased tissue.

A MALDI-MSI process comprises two steps: preparation of the sample including deposition of the matrix which may also comprise, if necessary, extraction of the analyte from the sample such as a tissue section, followed by ionization and desorption of the analyte by intense short pulses of laser light. The matrix provides for absorption of energy from the laser light to desorb the analyte and promotes ionization.

As discussed above, a major challenge in MALDI mass spectrometry and MALDI mass spectrometry imaging is detection of proteins of high molecular mass.

The present invention provides for a new method for preparing samples, in particular tissue sections, allowing the efficient extraction as well as the subsequent ionization, desorption and detection of high molecular mass proteins. The present invention provides a method for preparing a sample wherein the method comprises forming homogenous micro-crystals of matrix and proteins on the sample such as a biological tissue section. This method provides for efficient extraction, ionization and desorption of high molecular mass proteins which are detectable by MALDI based mass spectrometry (MALDI-MS) or MALDI based mass spectrometry imaging (MALDI-MSI) with high intensity.

The present invention is related to a method for the detection of at least one protein in a biological sample by Matrix Assisted Laser Desorption Ionization Mass Spectrometry (MALDI-MS) comprising the following steps:
a) Applying a solution comprising an extraction solvent selected from TFE (2,2,2-trifluoroethanol) and HFIP (1,1,1,3,3,3-hexaftuoro-2-propanol) onto the surface of the biological sample;
b) Applying a first SA MALDI matrix onto the surface of the biological sample and obtaining a first crystallized matrix on the surface of the biological sample;
c) Applying a second MALDI matrix onto the surface of the biological sample to solubilise the first crystallized matrix obtained in the previous step and re-crystallize both the first and the second matrix to obtain a crystallized matrix on the surface of the biological sample;
d) Ionization and desorption of proteins from the biological sample;
e) Analysis and detection of at least one protein by Mass Spectrometry.

The methods of the present invention are preferably *in vitro* methods.

The analytes detected by the methods of the present invention are polypeptides and proteins. The methods of the present invention are a particularly useful for the detection of polypeptides and proteins having a high molecular mass. Any protein including modified and/or labeled proteins may be detected by the methods of the present invention.

Advantageously, the methods of the present invention provide for detection of proteins having a high molecular weight or a high m/z by MALDI-MS and /or MALDI-MSI.

The methods of the present invention enable detection of proteins ranging from m/z 20 000 up to m/z 110 000.

In preferred embodiments, the methods of the present invention detect proteins having a m/z of at least 20 000, 30 000, 40 000, 50 000, 60 000 or 70 000.

Preferably, the methods of the present invention provide for detection both of proteins having a m/z below 20 000 and of proteins having a m/z above 20 000.

M/z refers to mass-to-charge ratio obtained by dividing the mass of an ion by the unified atomic mass unit and by its charge number.

The methods of the present invention enable detection of proteins ranging in molecular weight from 20 kDa up to 110kDa.

In preferred embodiments, the methods of the present invention detect proteins having a molecular weight of at least 20 000, 30 000, 40 000, 50 000, 60 000 or 70 000kDa.

Preferably, the methods of the present invention provide for detection both of proteins having a molecular weight below 20 000 and of proteins having a molecular weight above 20 000.

The methods of the present invention are also suitable to detect both polar and apolar proteins in biological samples such as tissue sections.

The term "sample" refers to any biological sample. In preferred embodiments, the sample is a biological tissue section or cell culture. The sample is usually immobilized onto a solid support. Solid supports or plates suitable for MALDI-MS are largely described in the literature. Any suitable support may be used in the methods of the present invention.

Proteins may be detected, localized and/or identified in any biological tissue section of interest. The biological tissue section preferably has a thickness comprised between 5 and 20µm. Any tissue or organism may be analyzed in the methods of the present invention. The tissue section is prepared according to known methods. Thin sections/slices are typically obtained from frozen tissues and applied onto glass plates. The tissue sections are then usually dried in a dessicator. The tissue sections immobilized onto a solid support may also be washed prior to further preparation of the sample for MALDI-MS.

The biological tissue section may be analysed by Matrix Assisted Laser Desorption Mass spectrometry Imaging in positive ion and/or negative ion reflector mode.

The term "detection" may refer both to localization and identification of the protein in the sample.

The methods of the present invention comprise an extraction step in which proteins are extracted from the biological sample. This extraction is performed by applying a solution comprising an extraction solvent selected from TFE (2,2,2-trifluoroethanol) and HFIP (1,1,1,3,3,3-hexafluoro-2-propanol) onto the surface of the biological sample. Any solution comprising TFE or HFIP suitable for the extraction of proteins from biological samples may be used in the methods of the present invention. Suitable solutions are described in the state of the art and known to the skilled technician.

In preferred embodiments, the solution applied onto the biological sample comprises TFE, water and TFA (Trifluoracetic acid).

Preferably, the solution comprises between 20% and 80% TFE. Preferably, the solution comprises between 5% and 25% TFA.

In other embodiments, the solution applied onto the biological sample is composed of HFIP. However, HFIP is both toxic and corrosive and it is therefore preferred to apply HFIP directly with the MALDI matrix as described below.

The solution comprising the extraction solvent is applied onto the biological sample i.e. the biological tissue section by any appropriate method. Such methods include direct dispensation to the surface of the sample with a pipette, spraying or printing small amounts of matrices by way of a chemical inkjet printer. The solution may be applied onto the whole surface of the biological sample or may be applied in a defined pattern of micro-spots according to known methods. If the solution is deposited by micro-spotting then the minimal quantity will be about 10 nL per spot position to cover. If the solution is deposited by micro-spraying the minimum amount of solution is 200 µL. If the solution is deposited by micropipette the minimum volume is then 5 µL.

The following steps in the sample preparation lead to deposition of the matrix and suitable crystallization of the matrix onto the biological sample. Crystallization of the matrix onto the biological sample leads to co-crystallizaton of the matrix and of the proteins which have been extracted in the previous step. The inventor's have surprisingly found that adequate crystallization of the matrix requires deposition of a first matrix, crystallization of the first matrix, deposition of a second matrix, solubilisation of said first matrix upon deposition of the second matrix and finally re-crystallization of the first and second matrix on the surface of the biological sample to obtain a crystallized matrix.

The methods of the present invention comprise applying a first MALDI matrix onto the surface of the biological sample and obtaining a first crystallized matrix on the surface of the biological sample. The inventors have observed that this first crystallized matrix is formed of heterogeneous crystals having a large size. This crystallized matrix is not suitable for efficient ionization, desorption and detection of proteins by MALDI-MS.

The methods of the present invention comprise applying a second MALDI matrix onto the surface of the biological sample to solubilise the first crystallized matrix obtained in the previous step and re-crystallize both the first and the second matrix to obtain a crystallized matrix on the surface of the biological sample. The matrix which is formed after solubilisation of the first matrix and crystallization of both the first and the second matrix exhibits homogenous micro-crystals which provide for efficient ionization, desorption and detection of proteins by MALDI-MSI.

The following steps of the methods of the present invention use classical methods for the ionization/desorption of proteins from the biological sample and for analysis/detection of at least one protein by Mass Spectrometry. These steps are carried out using any suitable MALDI-MSI technique. Ionization and desorption are classically performed by bombarding the sample with a laser beam. Subsequent, analysis and detection of at least one protein by Mass Spectrometry is preferably performed by TOF (time of flight)-Mass Spectrometry. As described above, the detector is adavantageously a high mass detector such as a COVALX™ detector.

As mentioned above, HFIP is both toxic and corrosive and it may therefore be advantageous to perform simultaneously steps a) and step b) of the method of the present invention. A single composition comprising both HFIP and the first MALDI matrix is applied onto the surface of the biological sample. This step provides for both extraction of the proteins from the sample and for the formation of a first crystallized matrix on the surface of the sample.

In preferred embodiments, the methods of the invention therefore comprise a step wherein step a) and step b) are performed simultaneously by applying a composition onto the surface of the biological sample comprising both HFIP and a first MALDI matrix.

Preferably, this composition comprises both HFIP and SA (Sinapinic acid), more preferred the composition consists of 20mg/ml SA in HFIP.

The term "matrix" refers to a material which generates matrix-embedded analyte molecules (i.e. proteins) that are successfully desorbed by laser irradiation and ionized from the solid phase. The term "matrix" is used both to refer to matrix solutions (matrix in appropriate solvent) and crystallized matrix obtained after evaporation of the solvent. The matrix usually consists of molecules of low molecular weight to provide for vaporization but large enough not to evaporate during sample preparation or while standing in the spectrometer. Matrices also have a strong optical absorption in the UV range, so that they rapidly and efficiently absorb the laser irradiation. In classical MALDI, the matrix solution is mixed with the sample or applied onto the sample. The organic solvent allows hydrophobic molecules to dissolve into the solution, while the water allows for watersoluble (hydrophilic) molecules to do the same. Then, the solvents vaporize, leaving only the re-crystallized matrix, but now with protein molecules spread throughout the crystals. The matrix and the analyte are said to be co-crystallized in a MALDI spot.

Any MALDI matrix suitable for the analysis and detection of proteins by MALDI-MS may be used in the methods of the present invention. The most commonly used matrix in protein analysis is sinapinic acid (SA). Other MALDI matrices can also be used such as 2,5-DHB (2,5-dihydroxybenzoic acid), SA/ANI (Aniline), SA/3AP (3-acetylpyridine), SA/DIENI (Diethylaniline) and SA/DANI (Dimethylaniline).

The preferred MALDI matrix for the detection of proteins is the SA matrix. Any SA matrix solution suitable for MALDI-MS may be used in the methods of the present invention. A solution of SA matrix is usually prepared in a mixture of highly purified water and of an organic solvent (normally acetonitrile (ACN) or ethanol). Normally some acid such as Trifluoroacetic acid (TFA) may also be added. SA is dissolved in the solvent and the concentration of SA in the matrix solution is in the range between 10mg/ml and 50 mg/ml.

In preferred embodiments, the first SA matrix in step b) and/or the second SA matrix in step c) comprises SA, ethanol, water and TFA. Preferably, the SA matrix comprises 80% to 90% ethanol and 10% to 20% TFA 0.1% in water.

In preferred embodiments, the first SA matrix in step b) and/or the second SA matrix in step c) comprises SA, ACN (acetonitrile), water and TFA. Preferably, the SA matrix comprises between 50% to 90% ACN and between 10% to 50% TFA 0.1% in water.

Even more preferred, the first SA matrix in step b) comprises SA, ethanol, water and TFA and the second SA matrix in step c) comprises SA, ACN (acetonitrile), water and TFA.

The concentration of SA in the matrix solution is usually comprised between 10mg/ml and 50mg/ml, preferably, the first SA matrix and the second SA matrix comprise 20mg/ml SA.

The matrix or the matrix solution is applied onto the biological sample i.e. the biological tissue section by any appropriate method. Such methods include direct dispensation to the surface of the sample with a pipette, spraying or printing small amounts of matrices by way of a chemical inkjet printer. The solution may be applied onto the whole surface of the biological sample i.e. tissue section or may be applied in a defined pattern of micro-spots according to known methods.

If the solution is deposited by micro-spotting then the minimal quantity will be about 10 nL per spot position to cover. If the solution is deposited by micro-spraying the minimum amount of solution is 200 µL. If the solution is deposited by micropipette the minimum volume is then 5 µL.

When the tissue sections are prepared according to the method of the present invention, high molecular weight proteins are detected by MALDI-MS or MALDI-MSI. The methods of the present invention are particularly suitable for the detection of apolar proteins and of proteins having a high molecular weight.

The invention also relates to methods for detecting in a single biological sample both polar or small proteins having a molecular weight below 20kDa and apolar or high molecular weight proteins having a molecular weight above 20kDa.

In these methods, the biological sample is first prepared by classical methods. Typically a SA matrix is deposited onto the biological sample and this sample is then subjected to MALDI-MS or MALDI-MSI. The first MALDI-MS analysis provides for detection and localization of small and polar proteins. The SA matrix is then removed from the biological sample which is further processed using the methods of the present invention specifically adapted for the detection of high molecular weight and apolar proteins.

Advantageously, small and larger proteins are detected in the same biological sample thanks to the methods of the present invention.

The invention is related to methods for the detection of proteins in a biological sample by MALDI-MS comprising the following steps:
a) Applying a solution comprising an extraction solvent selected from TFE (2,2,2-trifluoroethanol) or HFIP (1,1,1,3,3,3-hexafluoro-2-propanol) onto the surface of the biological sample;
b) Applying a first MALDI matrix onto the surface of the biological sample and obtaining a first crystallized matrix on the surface of the biological sample;
c) Applying a second MALDI matrix onto the surface of the biological sample to solubilise the first crystallized matrix obtained in the previous step and re-crystallize both the first and the second matrix to obtain a crystallized matrix on the surface of the biological sample;
d) Ionization and desorption of proteins from the biological sample;
e) Analysis and detection of at least one protein by Mass Spectrometry.

The invention is also related to methods for the detection of proteins in a biological sample by MALDI-MS comprising the following steps :
a) Applying a MALDI matrix onto the surface of the biological sample and obtaining a crystallized matrix on the surface of the biological sample;
b) Ionization and desorption of proteins from the biological sample;
c) Analysis and detection of proteins by Mass Spectrometry;
d) Removing the MALDI matrix applied in step a);
e) Applying a solution comprising an extraction solvent selected from TFE (2,2,2-trifluoroethanol) or HFIP (1,1,1,3,3,3-hexafluoro-2-propanol) onto the surface of the biological sample;
f) Applying a first MALDI matrix onto the surface of the biological sample and obtaining a first crystallized matrix on the surface of the biological sample;
g) Applying a second MALDI matrix onto the surface of the biological sample to solubilise the first crystallized matrix obtained in the previous step and re-crystallize both the first and the second matrix to obtain a crystallized matrix on the surface of the biological sample;
h) Ionization and desorption of proteins from the biological sample;
i) Analysis and detection of at least one protein by Mass Spectrometry.

Advantageously, steps a) to c) provide for the detection of at least one protein having a molecular weight below 20kDa and steps d) to e) or d) to i) provide for the detection of at least one protein having a molecular weight above 20kDa.

Any MALDI matrix suitable for the detection of proteins by MALDI-MS may be used in step a), preferably a SA matrix is used. SA matrices for use in step a) are described above, preferably the SA matrix in step a) comprises SA, ACN, water and TFA.

Other matrices include 2,5-DHB (2,5-dihydroxybenzoic acid), SA/ANI (Aniline), SA/3AP (3-acetylpyridine), SA/DIENI (diethylaniline) and SA/DANI (dimethylaniline).

Removal of the matrix in step d) is performed according to known methods; preferably the MALDI matrix is removed in a bath of methanol and water or ethanol and water. Typically the solid support carrying the biological sample i.e. tissue section is incubated for one minute in a bath of methanol and water (1:1, v/v) or ethanol and water (1:1, v/v).

Steps e) to i) are performed as described above.

### FIGURES

**Figure 1**: MALDI MSI of peptides/proteins in the positive linear mode recorded from a rat brain tissue section prepared by automatic spraying of SA 10mg/mL in AcN/aqueous TFA0.1% (7:3, v/v) **(a)** Optical image of the tissue section, **(b)** overlaid molecular images of ions at m/z 6°717, 7°538 and 14°123, **(c)** molecular images reconstructed for different ions detected at m/z 6717, m/z 7063, m/z 7538, m/z 8466, m/z 14123 and m/z 18407.
**Figure 2**: MALDI MS spectra acquired in positive linear mode after deposition of solution containg SA in ACN/TFA0.1% (7:3, v/v) from a rat brain tissue section **(a)** untreated versus **(b)** treated with 3 successive bathes of cold acetone, cold EtOH 95% and chloroform tissue sections.
**Figure 3**: MS spectra acquired in positive linear mode from a rat brain tissue section after deposition of SA in ACN/TFA0.1% (7:3 v:v) as matrix solution. **(a)** for a MCP detector and **(b)** a high mass detector.
**Figure 4**: MS spectra acquired in positive linear mode from a rat brain tissue section after **(a)** TFE treatment and **(b)** HFIP treatment.
**Figure 5**: MS spectra acquired in positive linear mode from a rat brain tissue section after deposition of **(a)** SA in ACN/TFA0.1% (7:3, v/v), **(b)** SA in ACN/TFA 0.1% (7:3, v/v) followed by removing of the matrix prior to TFE treatment and **(c)** SA in ACN/TFA 0.1% (7:3, v/v) followed by removing of the matrix prior to HFIP treatment.
**Figure 6**: MS spectra acquired in positive linear mode from an ovarian cancer tissue after deposition of **(a)** SA in ACN/TFA 0.1 % (7:3, v/v) and **(b)** SA in ACN/TFA 0.1 % (7:3, v/v) followed by removing of the matrix prior to HFIP treatment.
**Figure 7**: MS spectra acquired in positive linear mode from a mouse brain tissue section after HFIP treatment with **(a)** the MCP detector and **(b-c)** the high mass detector detector.

### EXAMPLES

### 1) Materials and methods

### Materials

Sinapinic acid (SA), 1,1,1,3,3,3-Hexafluoro-2-propanol (HFIP), Trifluoracetic acid (TFA), 2,2,2-Trifluoroethanol (TFE), Ethanol (EtOH), Acetone, Acetonitrile (ACN), Chloroform, Water Chromasolv plus for HPLC (H₂O) were purchased from Sigma-Aldrich (Saint Quentin Fallavier, France).

### Samples

**Rat brains**. Adult male Wistar rats weighing 250-350g (animal use accreditation by the French ministry of the agriculture N° 04860) maintained under standard care were used. Animals were sacrificed by decapitation and immediately dissected to remove the brain. Frozen sections of 15 µm were performed on a cryostat and immediately transferred onto the MALDI stainless steel plate.

**Ovarian Biopsies**. Tissues, ascites and cyst fluids were obtained, with informed consent and institutional review board approval (CCPPRBM Lille: CP 05/83), from patients undergoing any ovarian tumour resection at Hospital Jeanne de Flandre (Lille, France). Patient information was collected, including gender, age, treatment received before and after surgery, extent of surgery, current status (alive, alive with progressive disease, deceased, and cause of death), and survival from the time of original pathologic diagnosis. Samples were collected at the time of surgery, immediately frozen, and stored at -80°C until analysis.

### Tissue preparation

**Animal brains & ovarian biopsies**. Thin 10-12 µm tissue sections were obtained from frozen rat/mouse brains and ovarian biopsies using a cryostat Leica CM1510S (Leica Microsystems, Nanterre, France) and applied onto ITO-coated conductive glass slides (Bruker Daltonics, Bremen, Germany). For ovarian biopsies, Histopathology diagnoses were performed by a pathologist, blinded to the original clinical diagnosis, from subsequent H&E-stained sections. Tissue sections were submitted to different washing steps in order to get rid of salts and abundant lipids. Tissue section were first immersed in a bath of cold acetone(18) during 30 seconds followed by a bath with cold EtOH 95%(19) during 30 seconds. In a last step, tissues were washed using chloroform(18) by immersion in chloroform for 1 minute.

### MALDI MSI of proteins

5 mL of a solution containing 10mg/mL of SA in AcN/aqueous TFA 0.1% (7:3, v/v) was prepared. The solution was dropped of using an automatic sprayer (ImagePrep, Bruker Daltonics, Bremen, Germany) which creates matrix aerosol by vibrational vaporization under controlled conditions. This system monitors the different steps of matrix deposition, i.e. extraction, drying and allows controlling matrix layer thickness. Molecular images were acquired on an UltraFlex II MALDI-TOF/TOF instrument (Bruker Daltonics, Bremen, Germany) equipped with a Multiple Channel Plate (MCP) detector and on an AutoFlex III MALDI-TOF instrument (Bruker Daltonics, Bremen,Germany) equipped both with a MCP and a high molecular mass detector (CovalX, Switzerland). Both instruments are equipped with a Smartbeam laser having a repetition rate up to 200 Hz and are controlled by FlexControl 3.0 (Build 158) software (Bruker Daltonics, Bremen, Germany). Images were performed in the positive linear mode and MALDI MS spectra were acquired in the 4°000-40°000 and 4°000-90°000 m/z range respectively with the UltraFlex II and the AutoFlex III. A total of 1000 laser shots were averaged at each spot at a laser frequency of 200 Hz. The images were saved and reconstructed using FlexImaging 2.1 (Build 15) (Bruker Daltonics, Bremen, Germany).

### High mass proteins measurements with MCP or CovaIX detectors

High mass proteins measurements were performed from the tissue sections used for imaging in standard conditions after removing matrix from the tissue section with a bath of MeOH/water (1:1, v/v) for 1 min. Two procedures are then performed on the tissue section. First procedure consists in manually dropping of, using a micropipette, a solution containing 10mg/mL of SA in pure HFIP followed by deposition of a solution containing 20mg/mL of SA in AcN/ aqueous TFA 0.1 % (7:3, v/v). The second procedure consists in depositing manually using a micropipette a solution of TFE/ aqueous TFA 20% (1:1, v/v) followed by deposition of a solution containing 20mg/mL of SA in EtOH/ aqueous TFA 0.1% (9:1, v/v) and then a solution containing 10mg/mL of SA in AcN/ aqueous TFA 0.1% (7:3, v/v).

### High mass proteins MALDI MSI using Automatic spraying

A solution containing 10mg/mL of SA in TFE/aqueous TFA 10% (1:1, v:v) was first deposited on the whole rat brain tissue section according to a base of 20 spray cycles of 1.5 seconds. Subsequently, a solution containing 10mg/mL of SA in EtOH/ aqueous TFA 0.1% (9:1, v/v) was then sprayed according to the method recommended by Bruker. Finally, a solution containing 5mg/mL of SA in AcN/aqueous TFA 0.1% (7:3, v/v) was sprayed on top of the previous layer according to the same deposition parameters.

### High mass proteins MALDI MSI using Automatic micro-spotting

On a rat brain tissue section, 15nL of a solution containing 10mg/mL of SA in TFE/aqueous TFA 10% (1:1, v/v) was deposited using a high accurate position Chemical Inkjet Printer CHIP-1000 (Shimadzu, Kyoto, Japan) following a raster constituted by 4x4 spots in order to control the wetness. 10 nL of a solution containing 10mg/mL of SA in EtOH/ aqueous TFA 0.1% (9:1, v/v) was then deposited on the same spots followed by the deposition of 15nL of a solution containing 5mg/mL of SA in AcN/aqueous TFA 0.1% (7:3, v/v).

### 2) Comparative example: MALDI MSI of proteins after extraction procedures

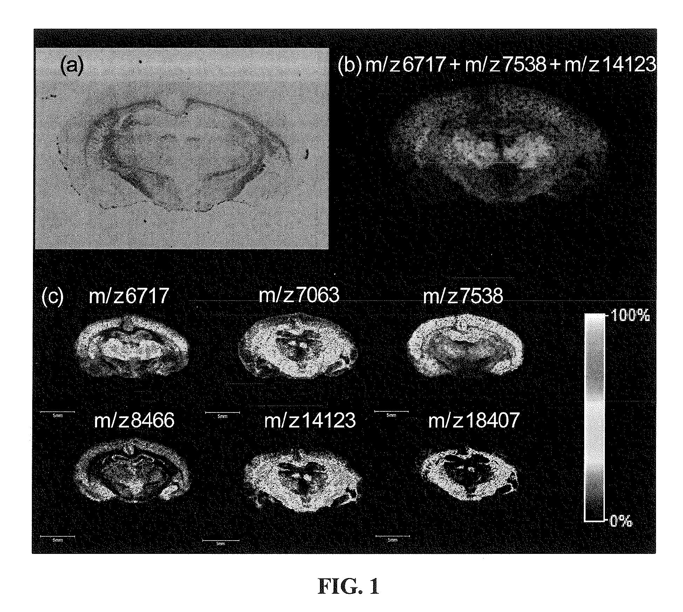

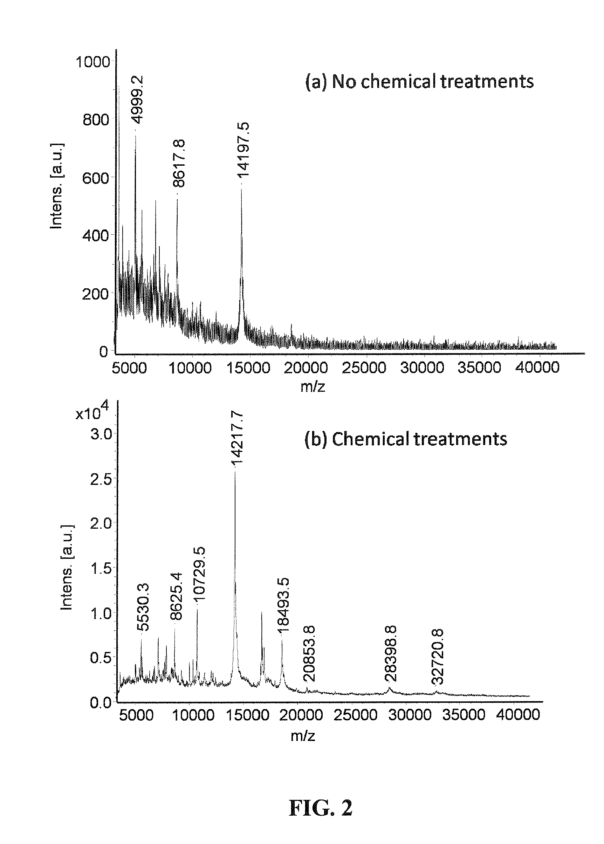

In MALDI MS as well as in MALDI MSI, proteins are generally analysed using SA as matrix in polar solvent such as AcN/aqueous TFA0.1%. **Figure 1** presents classical protein measurements directly from rat brain tissue section using automatic spraying of the matrix. In this study, a solution of SA 10mg/mL in AcN/aqueous TFA0.1% (7:3, v:v) was used for spraying. These conditions are more or less the classical conditions of preparation for SA in MALDI. By using this matrix solution, in combination with automatic spraying, a thin homogenous layer of small crystals of SA was generated. The quality of the preparation reflects on the images and mass spectra with very specific profiles were detected according to the location on the surface of the tissue section (**Figures 1b & 1c**). However, with such preparation protocols if highly specific and resolved molecular images are obtained, only a restricted number of proteins on a restricted mass range are observed. Most of the proteins observed with reasonable signals are generally below 25°000-30°000 Da. Thus, in order to access higher mass proteins, chemical treatments were performed. After chemical treatment with acetone, chloroform and EtOH, intense signals of proteins were detected with specific localization. Absolutely no delocalization of analytes was observed outside of the tissue section (**Figure 2**). It was already shown in a previous studies that chemical treatments using organic solvents such as chloroform (18), or other solvents such as EtOH (19, 20) provide spectra with higher intensity and provide detection of a larger number of proteins signals. **Figure 2a** shows the spectrum obtained directly from a rat brain tissue section without any chemical treatment. It clearly appears that few proteins are detected with a relatively low intensity and signal/noise ratio. On the contrary, spectra acquired after treatment with organic solvents from a consecutive rat brain tissue section show a very high number of intense signals of proteins up to m/z 20°000 and few protein signals with lower intensity in the 20°000-32°000 m/z range (**Figure 2b**). Such an effect is to be attributed to the removal by the solvent treatments of salts and small organic compounds such as abundant lipids that create suppression effects. Nevertheless, as shown in **Figure 2b**, even after such chemical treatments, protein signals above m/z 20°000 are very difficult or impossible to detect.

### 3) Comparative example: Use of a high mass detector

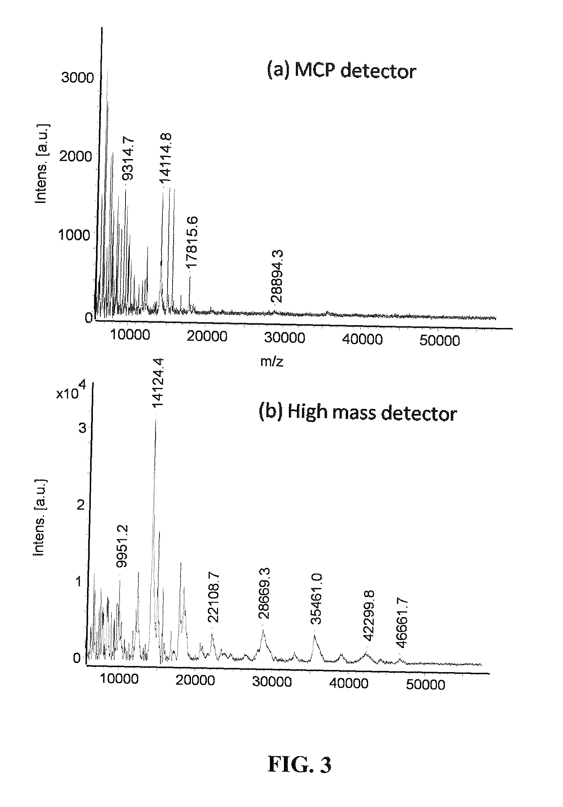

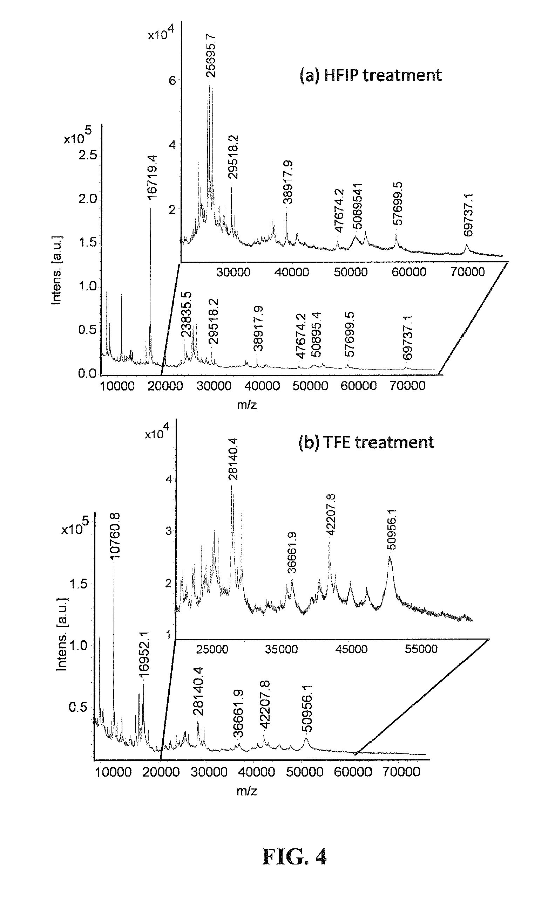

Whole surface of mouse brain tissue sections were covered with a matrix solution containing 20mg/mL of SA in AcN/aqueous TFA 0.1 % (7:3, v/v). Dried sections were subjected to a direct analysis using both MCP and CovaIX detector coupled onto an AutoFlex III MALDI-TOF instrument. The spectra, presented in **Figure 3**, were obtained by accumulation of 10°000 shots over the whole surface of the tissue section. The MS spectra recorded from the MCP detector shows many signals up to m/z 20°000 and only very few peaks with low intensity above 20°000 up to 35°000. No signals are observed above 35°000 (**Figure 3a**). On the contrary, with the high mass detector, different proteins are easily detected up to m/z 50°000 (**Figure 3b**) proving that difficulties in observing high mass proteins are not only due to solvent extraction efficiency from the tissue section but also due to lack of sensitivity in the detection. Images of proteins were then performed from mouse brain tissue sections after washing treatments (cold acetone, chloroform and cold EtOH 95%) and SA matrix deposition by automatic spraying using both the MCP and the high mass detector. As expected, mass spectra recorded with the high mass detectors were showing different signals above m/z 30°000 with a good intensity. Molecular images of three of these proteins are reported Figure 4. The first protein showing a signal at m/z 69°000 is localized in the third ventricule, the protein associated to the signal at m/z 46°850 is found in the cerebral cortex and the signal at m/z 44500 corresponds to a protein which is distributed in the cerebral nucleus and medulla oblongata of rat brain. Without any chemical treatments, the signals associated to these proteins are not detected and the molecular images can not be generated proving the efficiency of high mass detection for MSI experiments.

However, as shown in **Figure 3b**, the spectral resolution of proteins detected is worse with the high mass detector than with the commonly employed MCP detector. This loss of spectral resolution is to be attributed to the post-acceleration of the small ions after the first conversion step. Thus, high mass detection is only of interest above a certain m/z of at least a few thousand. We must also notice that the signals observed with the high mass detector have a higher intensity than with the MCP detector. However, only a few peaks that are detected with the high mass detector were not already observed with the MCP. This means that improving the detection is a real necessity for improving MALDI MSI oh high molecular masses but is not a sufficient condition. It is, thus, necessary to develop different treatments to improve extraction, incorporation into matrix crystal and ionization efficiency of these molecules.

### 4) Comparative example: chemical treatment for detection of high mass proteins

First tests were performed by adding manually using a micropipette an aqueous TFA 10% solution on the whole surface of a rat brain tissue section in order to create charge repulsion between proteins. A matrix solution containing 20mg/mL of SA in AcN/aqueous TFA 0.1% (7:3, v/v) was then added on the same surface before analysis. However, a detailed examination of the recorded MS spectra with this sample preparation method reveals that no improvements are generated.

In a second study, detergents were then used to provoke dispersion of hydrophobic molecules and protein unfolding. This was obtained by addition of an aqueous TFA 10% solution containing 1% of octyl B-D-glucopyranoside (OG) as detergent. Detergents are often a problem for MS analysis although, OG is a non ionic detergent that was previously shown to be compatible with MSI (25). However, recorded MS spectra using this second protocol do not show any improvement compared to those obtained without any treatment.

Thus, it seems that proteins are still difficult to solubilize, even after disruption of hydrophobic interactions, preventing their co-crystallization with the matrix and thus their desorption/ionization.

### 5) Procedures for detection of high molecular mass proteins

Two types of procedure were then elaborated to improve the detection of high mass proteins.

The first procedure consists in the deposition of a solution of aqueous TFA20%/TFE (1:1, v/v). In this solvent mixture, TFA is used to decrease the pH in order to promote the creation of charge repulsions required for the rupture of non covalent interactions and subsequent unfolding of polypeptides and separation of proteins. TFE is used to allow the extraction of separated and unfolded proteins and to create the hydrophobic environment required by the exposure of apolar groups of proteins to the SA crystals. Co-crystallization of analytes with SA is an important element for high mass proteins desorption/ionization. Since SA is not soluble in TFE, another solvent was added to dissolve the matrix. 10 µL of a solution containing 20mg/mL of SA in EtOH/aqueous TFA 0.1% (9:1, v/v) was then applied to trap the proteins dissolved by TFE. This leads to the formation of big crystals provoking an important background noise and a dramatic elevation of the baseline of the spectra. Thus, the procedure was completed by addition of a last solution containing 20mg/mL of SA in AcN/aqueous TFA 0.1% (7:3, v/v). This solution allows the re-solubilization of SA crystals leading to the formation of small white crystals containing the high mass proteins. MS spectra recorded from rat brain tissue sections after this procedure exhibit high intensity signals up to m/z 50°000 with good S/N. On the contrary, from the adjacent tissue section that has not received the chemical treatment no ions above m/z of 30°000 are observed (**Figure 4a**).

In order to reduce the number of steps needed for unfolding, extraction and incorporation in SA crystals of proteins and render it more compatible with the imaging mode, another strategy was developed. Thus, a solvent such as HFIP known for its extraction efficiency of hydrophobic proteins was considered. This solvent allows a complete solubilisation of SA and therefore a direct co-crystallisation with high mass proteins. The treatment of the most hydrophobic proteins is done by the addition of a layer of solution containing 20mg/mL of SA in pure HFIP followed by addition of a solution of 20mg/mL of SA in AcN/aqueous TFA0.1% (7:3, v/v) to re-solubilize the SA crystals formed with HFIP. This procedure leads to the detection of proteins up to m/z 70°000 with a high sensitivity and high reproducibility (**Figure 4b**). However, this strategy need to be handle with care due to the fact that HFIP can easily spread outside of the section.

### 6) Procedure for simultaneously detecting small polar proteins and high molecular mass proteins

Because high mass proteins require specific treatments using hydrophobic solvent to be detected, a strategy allowing for the detection of polar proteins prior to high mass detection was developed.

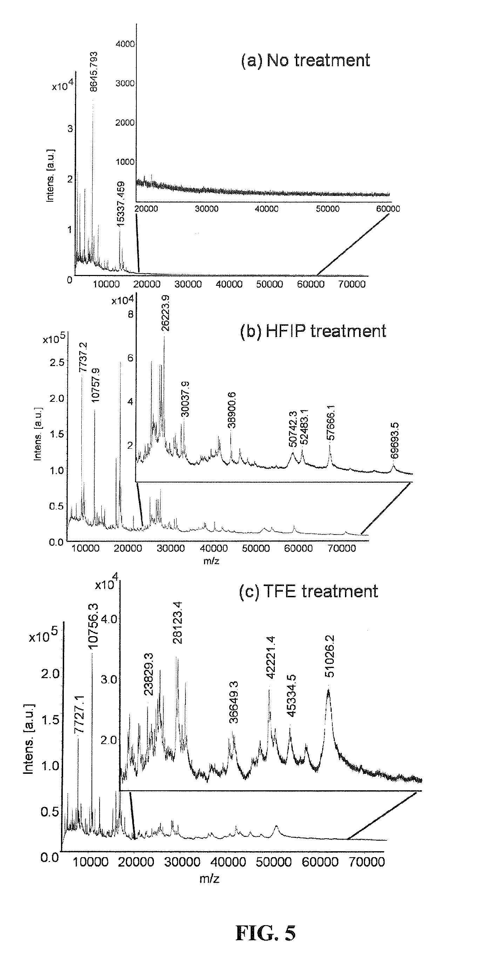

Rat brain tissue sections were treated using a solution containing SA in ACN/aqueous TFA 0.1% (7:3, v/v). This solution was manually deposited using a micropipette for profiling experiments or automatically sprayed for imaging. After analysis or imaging of the tissue section, the matrix was removed by a bath of MeOH/H2O (1:1, v/v) for 1 minute. Tissues were then completely dried of and submitted to treatments using TFE and HFIP. As shown in **Figure 5a**, before TFE/HFIP treatment, only low mass proteins up to m/z 20° 000 are detected as expected in the normal tissue analytical conditions. After removal of matrix and HFIP/TFE treatment, high mass proteins up to m/z 70°000 are detected with high sensitivity (**Figure 5b****-c**).

### 7) Sample Preparation for high mass proteins MALDI-MSI

The automated deposition of solutions for tissue treatment was tested to perform MSI of high mass proteins using either automated spraying or a high accurate position piezoelectric microspotter. Both procedures has previously described were tested at the microscale level. Automated spraying with **HFIP** failed because of the low viscosity this solvent. HFIP is hardly compatible with this deposition method as long as it flows out the instrument. HFIP was also not usable with micro-spotting since some parts equipping the piezoelectric head (nylon ring junction) would have been dissolved by this solvent leading to a irremediable clocking of the system. Thus, only the procedure including the use of TFE seems to be compatible with both automated deposition devices. For automated micro-spotting, 15 nL of each of the solutions were deposited according to a raster consisting of 4 by 4 spots in order to control the level of wetness for a better extraction of analytes. In the same manner, the TFE procedure was applied using the automated spraying device. Nevertheless, either using micro-spotting or spraying coating, only peaks with m/z <40°000 were observed (data not shown). These results show that the low amount of solution deposited by the automatic devices in order to limit analyte delocalization also limits the extraction efficiency of high mass proteins. Some further developments need to be done to access high mass proteins using automated deposition of these solutions for imaging.

### 8) Application in pathological proteomics: ovarian cancer

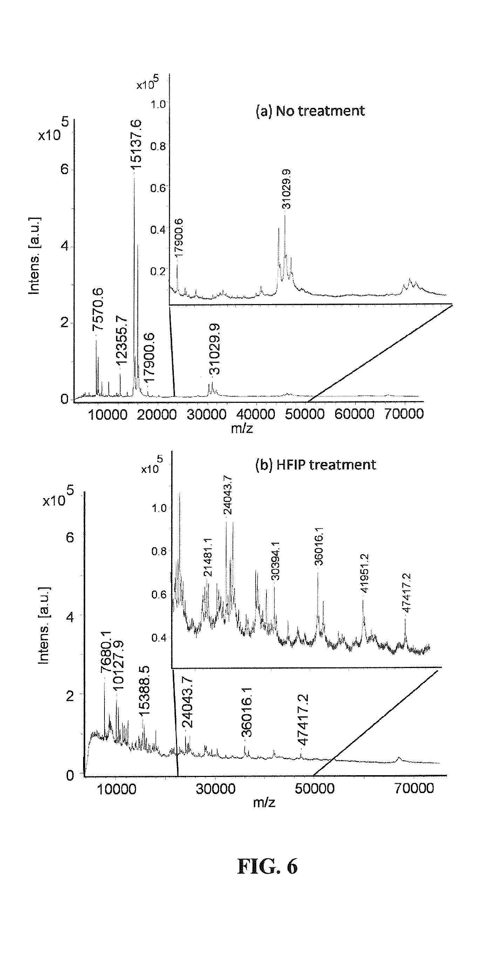

Ovarian cancer is now the fourth leading cause of cancer death among women in Europe and in the United States, with more than 22°000 new cases and 16°000 deaths reported each year in the U.S.A.(34). Despite advances in therapeutic management during the past decade, ovarian cancer remains the most lethal gynecological malignancy. Recently, our group applied MALDI MSI for biomarkers research of this pathology. MALDI MSI allowed the detection of different markers among which the Cter fragment of the immunoproteasome 11s (m/z 9744) as a biomarker for serous cancers (35). Here, one of the elaborated procedures was applied for the detection of high mass proteins from a cancerous ovarian tissue. On a tissue section from a cancerous biopsy, 10 µL of a solution containing 20mg/mL of SA in ACN/aqueous TFA 0.1% (7:3, v/v) was manually applied to perform a first analysis of polar proteins. As shown in **Figures 6a**, many proteins were detected u p to m/z 30°000 but only few ones above with this single layer of SA dissolved in classical solvents. This SA layer was then removed using a bath of Methanol/Water (1:1, v/v) and the HFIP procedure was then applied as described previously. **Figures 6b** show MS spectrum obtained after removal of the first SA layer and HFIP treatment. The number of peaks detected in the 20°000-50°000 m/z range has clearly increased using this chemical treatment procedure. One can easily imagine the usefulness of chemical treatments for biomarkers hunting using MSI..In this context, a comparison of molecular weights, as determined according to the proteins m/z measured by MALDI MSI using the high mass proteins unmasking and 2D gel proteomic procedure, suggest the detection of several already identified biomarkers (**Table 1**).

**Table 1: Molecular weight (Da) of ovarian cancer previously identified proteins and found in MALDI MSI after high mass protein procedure unmasking**

| **Protein Name** | **Molecular Mass (Da)** **Determined by MALDI MSI with high mass procedure** | **Molecular Mass (Da)** **Protein Identified in ovarian cancer** | **References** |
|---|---|---|---|
| Tetranectin (CAA45860) | 17°775.3 | 17°776 | (38-41) |
| Neutropil Gelatinase-associated lipocalin precursor | 22°576.4 | 22°571 | (42) |
| Plasma retinol-binding protein precursor | 22°9861 | 22°990 | (43) |
| Metalloproteinase inhibitor 1 precursor | 23°152.7 | 23°153 | (44) |
| Kallikrein 5 Precursor | 26°842.6 | 26°838 | (45) |
| Isoform 1 of Urokinase plasminogen activator surface receptor precursor | 36°940 | 36°949 | (46) (47) |

However, several unknown proteins were also detected by MALDI profiling. We recently developed a Bottom-Up strategy for MALDI MSI to identify directly from tissue sections unknown proteins. Thus, thanks to the chemical treatments done on cancerous regions of interest it will be now possible to establish a molecular diagnosis based directly on intact proteins. This is also promising for tissue classification using bioinformatics tools such as hierarchical clustering processing (36, 37).

### 9) Combination of chemical treatments and high mass detector

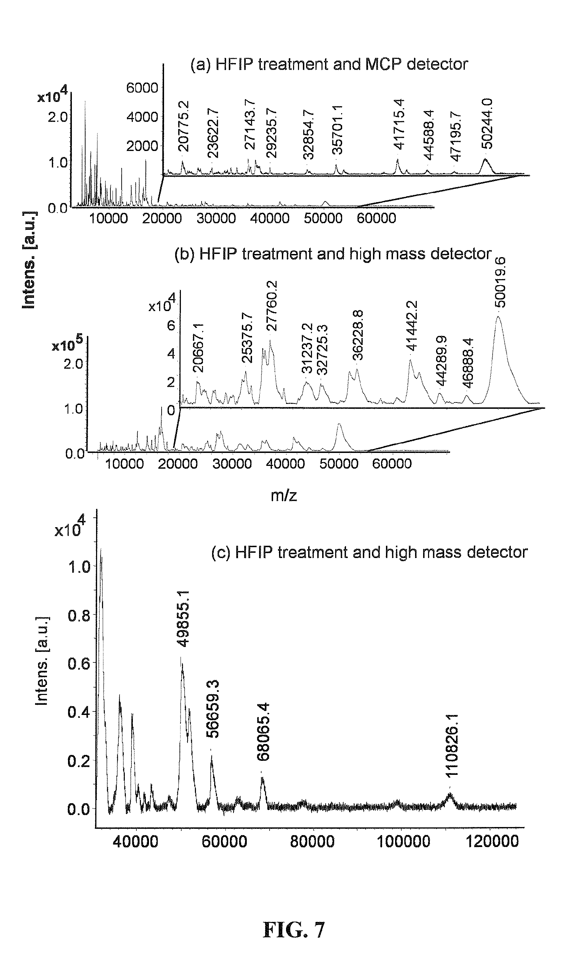

One of the chemical treatment procedures was then tested with the high mass detector. The HFIP procedure was applied on the whole surface of a mouse brain tissue section and spectra were acquired with both MCP and high mass detector. As shown in **Figure 7**, the spectrum acquired using the high mass detector presents peaks up to m/z 50°000, 50 fold increased in intensity (**Figure 7b**) than peaks observed with the conventional MCP detector (**Figure 6a**). Even though the resolution obtained with the high mass system is worse than the resolution achieved with the MCP detector, the high mass detector allows the observation of high mass proteins up to about m/z 110°000 (**Figure7c**). Thus, this procedure can easily be used for profiling assays of over 50 kDa proteins using high mass detection with a high sensitivity. This can be achieved after conventional imaging experiments using conventional preparation and detection on the same tissue section after removal of the first matrix layer and chemical treatments.

### REFERENCES

### Patent documents cited in the description

WO2009/086642

### Non-patent literature cited in the description

1. Reyzer, M. L., and Caprioli, R. M. (2007) MALDI-MS-based imaging of small molecules and proteins in tissues. Curr Opin Chem Biol 11, 29-35.
2. Rubakhin, S. S., Jurchen, J. C., Monroe, E. B., and Sweedler, J. V. (2005) Imaging mass spectrometry: fundamentals and applications to drug discovery. Drug Discov Today 10, 823-837.
3. Chaurand, P., Norris, J. L., Cornett, D. S., Mobley, J. A., and Caprioli, R. M. (2006) New developments in profiling and imaging of proteins from tissue sections by MALDI mass spectrometry. J Proteome Res 5, 2889-2900.
4. Chaurand, P., Sanders, M. E., Jensen, R. A., and Caprioli, R. M. (2004) Proteomics in diagnostic pathology: profiling and imaging proteins directly in tissue sections. Am J Pathol 165, 1057-1068.
5. Chaurand, P., Schwartz, S. A., and Caprioli, R. M. (2002) Imaging mass spectrometry: a new tool to investigate the spatial organization of peptides and proteins in mammalian tissue sections. Curr Opin Chem Biol 6, 676-681.
6. Fournier, I., Wisztorski, M., and Salzet, M. (2008) Tissue imaging using MALDI-MS: a new frontier of histopathology proteomics. Expert Rev Proteomics 5, 413-424.
7. Franck, J., Arafah, K., Elayed, M., Bonnel, D., Vergara, D., Jacquet, A., Vinatier, D., Wisztorski, M., Day, R., Fournier, I., and Salzet, M. (2009) MALDI IMAGING: State of the art technology in clinical proteomics. Mol Cell Proteomics.
8. Burnum, K. E., Cornett, D. S., Puolitaival, S. M., Milne, S. B., Myers, D. S., Tranguch, S., Brown, H. A., Dey, S. K., and Caprioli, R. M. (2009) Spatial and temporal alterations of phospholipids determined by mass spectrometry during mouse embryo implantation. J Lipid Res.
9. Dreisewerd, K., Lemaire, R., Pohlentz, G., Salzet, M., Wisztorski, M., Berkenkamp, S., and Fournier, I. (2007) Molecular profiling of native and matrix-coated tissue slices from rat brain by infrared and ultraviolet laser desorption/ionization orthogonal time-of-flight mass spectrometry. Anal Chem 79, 2463-2471.
10. Woods, A. S., Wang, H. Y., and Jackson, S. N. (2007) A snapshot of tissue glycerolipids. Curr Pharm Des 13, 3344-3356.
11. Sugiura, Y., Konishi, Y., Zaima, N., Kajihara, S., Nakanishi, H., Taguchi, R., and Setou, M. (2009) Visualization of the cell-selective distribution of PUFA-containing phosphatidylcho lines in mouse brain by imaging mass spectrometry. J Lipid Res.
12. Murphy, R. C., Hankin, J. A., and Barkley, R. M. (2009) Imaging of lipid species by MALDI mass spectrometry. J Lipid Res 50 Suppl, S317-322.
13. Leinweber, B. D., Tsaprailis, G., Monks, T. J., and Lau, S. S. (2009) Improved MALDI-TOF imaging yields increased protein signals at high molecular mass. J Am Soc Mass Spectrom 20, 89-95.
14. Grey, A. C., Chaurand, P., Caprioli, R. M., and Schey, K. L. (2009) MALDI Imaging Mass Spectrometry of Integral Membrane Proteins from Ocular Lens and Retinal Tissue (dagger). J Proteome Res 8, 3278-3283.
15. Redeby, T., Roeraade, J., and Emmer, A. (2004) Simple fabrication of a structured matrix-assisted laser desorption/ionization target coating for increased sensitivity in mass spectrometric analysis of membrane proteins. Rapid Commun Mass Spectrom 18, 1161-1166.
16. Redeby, T., Carr, H., Bjork, M., and Emmer, A. (2006) A screening procedure for the solubilization of chloroplast membrane proteins from the marine green macroalga Ulva lactuca using RP-HPLC-MALDI-MS. Int J Biol Macromol 39,29-36*.*
17. Lemaire, R., Stauber, J., Wisztorski, M., Van Camp, C., Desmons, A., Deschamps, M., Proess, G., Rudlof, I., Woods, A. S., Day, R., Salzet, M., and Fournier, I. (2007) Tag-mass: specific molecular imaging of transcriptome and proteome by mass spectrometry based on photocleavable tag. J Proteome Res 6, 2057-2067.
18. Lemaire, R., Wisztorski, M., Desmons, A., Tabet, J. C., Day, R., Salzet, M., and Fournier, 1. (2006) MALDI-MS direct tissue analysis of proteins: Improving signal sensitivity using organic treatments. Anal Chem 78, 7145-7153.
19. Seeley, E. H., Oppenheimer, S. R., Mi, D., Chaurand, P., and Caprioli, R. M. (2008) Enhancement of protein sensitivity for MALDI imaging mass spectrometry after chemical treatment of tissue sections. J Am Soc Mass Spectrom 19, 1069-1077.
20. Schwartz, S. A., Reyzer, M. L., and Caprioli, R. M. (2003) Direct tissue analysis using matrix-assisted laser desorption/ionization mass spectrometry: practical aspects of sample preparation. J Mass Spectrom 38, 699-708.
21. Thibault, D. B., Gillam, C. J., Grey, A. C., Han, J., and Schey, K. L. (2008) MALDI tissue profiling of integral membrane proteins from ocular tissues. J Am Soc Mass Spectrom 19, 814-822.
22. Ames, G. F., and Nikaido, K. (1976) Two-dimensional gel electrophoresis of membrane proteins. Biochemistry 15, 616-623.
23. Herskovits, T. T., Jaillet, H., and Gadegbeku, B. (1970) On the structural stability and solvent denaturation of proteins. II. Denaturation by the ureas. J Biol Chem 245, 4544-4550.
24. Horst, M. N., Basha, S. M., Baumbach, G. A., Mansfield, E. H., and Roberts, R. M. (1980) Alkaline urea solubilization, two-dimensional electrophoresis and lectin staining of mammalian cell plasma membrane and plant seed proteins. Anal Biochem 102, 399-408.
25. Djidja, M. C., Francese, S., Loadman, P. M., Sutton, C. W., Scriven, P., Claude, E., Snel, M. F., Franck, J., Salzet, M., and Clench, M. R. (2009) Detergent addition to tryptic digests and ion mobility separation prior to MS/MS improves peptide yield and protein identification for in situ proteomic investigation of frozen and formalin-fixed paraffin-embedded adenocarcinoma tissue sections. Proteomics 9, 2750-2763.
26. Redeby, T., and Emmer, A. (2005) Membrane protein and peptide sample handling for MS analysis using a structured MALDI target. Anal Bioanal Chem 381, 225-232.
27. Zhang, H., Lin, Q., Ponnusamy, S., Kothandaraman, N., Lim, T. K., Zhao, C., Kit, H. S., Arijit, B., Rauff, M., Hew, C. L., Chung, M. C., Joshi, S. B., and Choolani, M. (2007) Differential recovery of membrane proteins after extraction by aqueous methanol and trifluoroethanol. Proteomics 7, 1654-1663.
28. Wang, H., Qian, W. J., Mottaz, H. M., Clauss, T. R., Anderson, D. J., Moore, R. J., Camp, D. G., 2nd, Khan, A. H., Sforza, D. M., Pallavicini, M., Smith, D. J., and Smith, R. D. (2005) Development and evaluation of a micro- and nanoscale proteomic sample preparation method. J Proteome Res 4, 2397-2403.
29. Chertov, O., Biragyn, A., Kwak, L. W., Simpson, J. T., Boronina, T., Hoang, V. M., Prieto, D. A., Conrads, T. P., Veenstra, T. D., and Fisher, R. J. (2004) Organic solvent extraction of proteins and peptides from serum as an effective sample preparation for detection and identification of biomarkers by mass spectrometry. Proteomics 4, 1195-1203.
30. Ferro, M., Seigneurin-Berny, D., Rolland, N., Chapel, A., Salvi, D., Garin, J., and Joyard, J. (2000) Organic solvent extraction as a versatile procedure to identify hydrophobic chloroplast membrane proteins. Electrophoresis 21, 3517-3526.
31. Wang, W., Guo, T., Rudnick, P. A., Song, T., Li, J., Zhuang, Z., Zheng, W., Devoe, D. L., Lee, C. S., and Balgley, B. M. (2007) Membrane proteome analysis of microdissected ovarian tumor tissues using capillary isoelectric focusing/reversed-phase liquid chromatography-tandem MS. Anal Chem 79, 1002-1009.
32. Thompson, M. R., Chourey, K., Froelich, J. M., Erickson, B. K., Verberkmoes, N. C., and Hettich, R. L. (2008) Experimental Approach for Deep Proteome Measurements from Small-Scale Microbial Biomass Samples. Anal Chem 80, 9517-9525.
33. Reiersen, H., and Rees, A. R. (2000) Trifluoroethanol may form a solvent matrix for assisted hydrophobic interactions between peptide side chains. Protein Eng 13, 739-743.
34. Edwards, B. K., Brown, M. L., Wingo, P. A., Howe, H. L., Ward, E., Ries, L. A., Schrag, D., Jamison, P. M., Jemal, A., Wu, X. C., Friedman, C., Harlan, L., Warren, J., Anderson, R. N., and Pickle, L. W. (2005) Annual report to the nation on the status of cancer, 1975-2002, featuring population-based trends in cancer treatment. J Natl Cancer Inst 97, 1407-1427.
35. Lemaire, R., Menguellet, S. A., Stauber, J., Marchaudon, V., Lucot, J. P., Collinet, P., Farine, M. O., Vinatier, D., Day, R., Ducoroy, P., Salzet, M., and Fournier, I. (2007) Specific MALDI imaging and profiling for biomarker hunting and validation: fragment of the 11S proteasome activator complex, Reg alpha fragment, is a new potential ovary cancer biomarker. J Proteome Res 6, 4127-4134.
36. Deininger, S. O., Ebert, M. P., Futterer, A., Gerhard, M., and Rocken, C. (2008) MALDI imaging combined with hierarchical clustering as a new tool for the interpretation of complex human cancers. J Proteome Res 7, 5230-5236.
37. Walch, A., Rauser, S., Deininger, S. O., and Hofler, H. (2008) MALDI imaging mass spectrometry for direct tissue analysis: a new frontier for molecular histology. Histochem Cell Biol 130, 421-434.
38. Begum, F. D., Hogdall, C. K., Kjaer, S. K., Christensen, L., Blaakaer, J., Bock, J. E., Glud, E., Hoyer-Hansen, G., Ring-Larsen, H., and Hogdall, E. V. (2004) The prognostic value of plasma soluble urokinase plasminogen activator receptor (suPAR) levels in stage III ovarian cancer patients. Anticancer Res 24, 1981-1985.
39. Deng, X., Hogdall, E. V., Hogdall, C. K., Norgaard-Pedersen, B., Jorgensen, M., Nielsen, H., and Engelholm, S. A. (2000) The prognostic value of pretherapeutic tetranectin and CA-125 in patients with relapse of ovarian cancer. Gynecol Oncol 79, 416-419.
40. Hogdall, E. V., Hogdall, C. K., Tingulstad, S., Hagen, B., Nustad, K., Xu, F. J., Bast, R. C., and Jacobs, I. J. (2000) Predictive values of serum tumour markers tetranectin, OVX1, CASA and CA125 in patients with a pelvic mass. Int J Cancer 89, 519-523.
41. Lundstrom, M. S., Hogdall, C. K., Nielsen, A. L., and Nyholm, H. C. (2000) Serum tetranectin and CA125 in endometrial adenocarcinoma. Anticancer Res 20, 3903-3906.
42. Lim, R., Ahmed, N., Borregaard, N., Riley, C., Wafai, R., Thompson, E. W., Quinn, M. A., and Rice, G. E. (2007) Neutrophil gelatinase-associated lipocalin (NGAL) an early-screening biomarker for ovarian cancer: NGAL is associated with epidermal growth factor-induced epithelio-mesenchymal transition. Int J Cancer 120, 2426-2434.
43. Gericke, B., Raila, J., Sehouli, J., Haebel, S., Konsgen, D., Mustea, A., and Schweigert, F. J. (2005) Microheterogeneity of transthyretin in serum and ascitic fluid of ovarian cancer patients. BMC Cancer 5, 133.
44. Rauvala, M., Puistola, U., and Turpeenniemi-Hujanen, T. (2005) Gelatinases and their tissue inhibitors in ovarian tumors; TIMP-1 is a predictive as well as a prognostic factor. Gynecol Oncol 99, 656-663.
45. Diamandis, E. P., Borgono, C. A., Scorilas, A., Yousef, G. M., Harbeck, N., Dorn, J., Schmalfeldt, B., and Schmitt, M. (2003) Immunofluorometric quantification of human kallikrein 5 expression in ovarian cancer cytosols and its association with unfavorable patient prognosis. Tumour Biol 24, 299-309.
46. Pedersen, N., Schmitt, M., Ronne, E., Nicoletti, M. I., Hoyer-Hansen, G., Conese, M., Giavazzi, R., Dano, K., Kuhn, W., Janicke, F., and et al. (1993) A ligand-free, soluble urokinase receptor is present in the ascitic fluid from patients with ovarian cancer. J Clin Invest 92, 2160-2167.
47. Sier, C. F., Stephens, R., Bizik, J., Mariani, A., Bassan, M., Pedersen, N., Frigerio, L., Ferrari, A., Dano, K., Brunner, N., and Blasi, F. (1998) The level of urokinase-type plasminogen activator receptor is increased in serum of ovarian cancer patients. Cancer Res 58, 1843-1849.

## Claims

1. Method for the detection of at least one protein in a biological sample by Matrix Assisted Laser Desorption Ionization Mass Spectrometry (MALDI-MS) comprising the following steps:
a) Applying a solution comprising an extraction solvent selected from TFE (2,2,2-trifluoroethanol) and HFIP (1,1,1,3,3,3-hexafluoro-2-propanol) onto the surface of the biological sample;
b) Applying a first MALDI matrix onto the surface of the biological sample and obtaining a first crystallized matrix on the surface of the biological sample;
c) Applying a second MALDI matrix onto the surface of the biological sample to solubilise the first crystallized matrix obtained in the previous step and re-crystallize both the first and the second matrix to obtain a crystallized matrix on the surface of the biological sample;
d) Ionization and desorption of proteins from the biological sample;
e) Analysis and detection of at least one protein by Mass Spectrometry.

2. Method for detection of at least one protein in a biological sample by MALDI-MS according to claim 1 wherein the first and second MALDI matrix are selected from SA (Sinapinic acid), 2,5-DHB (2,5-dihydroxybenzoic acid), SA/ANI (Aniline), SA/3AP (3-acetylpyridine), SA/DIENI (Diethylaniline) and SA/DANI (Dimethylaniline).

3. Method for detection of at least one protein in a biological sample by MALDI-MS according to anyone of claims 1-2 wherein the first and second MALDI matrix are SA (Sinapinic acid) matrices.

4. Method for detection of at least one protein in a biological sample by MALDI-MS according to anyone of claims 1-3 wherein the solution applied in step a) comprises TFE, water and TFA (Trifluoracetic acid).

5. Method for the detection of at least one protein in a biological sample by MALDI-MS according to claim 1 wherein step a) and step b) are performed simultaneously by applying a composition onto the surface of the biological sample comprising both HFIP and a first SA matrix.

6. Method for the detection of at least one protein in a biological sample by MALDI-MS according to claim 5 wherein said composition applied onto the surface of the biological sample comprises 20mg/ml SA in HFIP.

7. Method for the detection of at least one protein in a biological sample by MALDI-MS according to anyone of claims 1 to 4, wherein the first MALDI matrix in step b) and/or the second MALDI matrix in step c) comprises SA, ethanol, water and TFA.

8. Method for the detection of at least one protein in a biological sample by MALDI-MS according to anyone of claims 1 to 4 and 7, wherein the first MALDI matrix in step b) and/or the second MALDI matrix in step c) comprises SA, ACN (acetonitrile), water and TFA.

9. Method for the detection of at least one protein in a biological sample by MALDI-MS according to anyone of claims 1-4 and 7-8, wherein the first MALDI matrix in step b) comprises SA, ethanol, water and TFA and the second MALDI matrix in step c) comprises SA, ACN (acetonitrile), water and TFA.

10. Method for the detection of at least one protein in a biological sample by MALDI-MS according to anyone of claims 1 to 9, wherein the first MALDI matrix and the second MALDI matrix comprise 20mg/ml SA.

11. Method for the detection of at least one protein in a biological sample by MALDI-MS according to anyone of claims 1 to 11, wherein analysis and detection of at least one protein by Mass Spectrometry in step e) is performed by TOF (time of flight)-Mass Spectrometry.

12. Method for the detection of proteins in a biological sample by MALDI-MS comprising the following steps :
a) Applying a MALDI matrix onto the surface of the biological sample and obtaining a crystallized matrix on the surface of the biological sample;
b) Ionization and desorption of proteins from the biological sample;
c) Analysis and detection of proteins by Mass Spectrometry;
d) Removing the MALDI matrix applied in step a);
e) Detection of at least one protein in the same biological sample by Matrix Assisted Laser Desorption Ionization Mass Spectrometry (MALDI-MS) by a method according to anyone of claims 1-11.

13. Method for the detection of proteins in a biological sample by MALDI-MS according to claim 12 wherein in step a) the MALDI matrix is selected from from SA (Sinapinic acid), 2,5-DHB (2,5-dihydroxybenzoic acid), SA/ANI (Aniline), SA/3AP (3-acetylpyridine), SA/DIENI (Diethylaniline) and SA/DANI (Dimethylaniline).

14. Method for the detection of proteins in a biological sample by MALDI-MS according to anyone of claims 12-13 wherein steps a) to c) provide for the detection of at least one protein having a molecular weight below 20kDa and wherein steps d) to e) provide for the detection of at least one protein having a molecular weight above 20kDa.

15. Method for the detection of proteins in a biological sample by MALDI-MS according to anyone of claims 12-14, wherein in step d) the MALDI matrix is removed in a bath of methanol and water.
